# FASCICULE DE BREVET EUROPEEN

(11) **EP 0 723 770 B1**
(45) Date de publication et mention de la délivrance du brevet: **29.10.1997**
(21) Numéro de dépôt: 95119754.0
(22) Date de dépôt: 14.12.1995
(51) Int. Cl.: A61K 7/06, A61K 7/48

(54) **Composition cosmétique à base de gomme de guar et de silicone oxyalkylène**
Kosmetische Zusammensetzung enthaltend Guargummi und oxyalkylierte Silicone
Cosmetic composition based on guar gum and oxyalkylated silicone

(30) Priorité: 27.01.1995 FR 9500983
(43) Date de publication de la demande: 31.07.1996
(73) Titulaire: L'OREAL, 75008 Paris (FR)
(72) Inventeur: Cretois, Isabelle, F-92110 Clichy (FR); Samain, Henri, F-91570 Bievres (FR)
(74) Mandataire: Tezier Herman, Béatrice

(56) Documents cités:
- EP-A- 0 562 638
- EP-A- 0 633 018
- FR-A- 2 707 163
- FR-A- 2 709 954
- GB-A- 2 173 515
- US-A- 5 188 756
- US-A- 5 217 652
- US-A- 5 344 643
- Dictionnaire CTFA pages 180 et 223

## Description

La présente invention concerne des compositions cosmétiques contenant, dans un milieu cosmétiquement acceptable, au moins une gomme de guar et au moins une silicone oxyalkylénée dans un rapport pondéral gomme de guar/silicone inférieur ou égal à 5.

L'invention a encore pour objet un procédé de traitement cosmétique non avant des matières kératiniques, telles que les cheveux ou les cils, caractérisé en ce qu'il consiste à appliquer sur les matières kératiniques une composition cosmétique telle que définie précédemment, puis à effectuer éventuellement un rinçage à l'eau.

On connaît déjà dans l'état de la technique des formulations permettant de fixer et de conditionner les cheveux. On a déjà utilisé dans ce but des compositions sous forme de gel qui sont généralement à base de polymères acryliques réticulés. Ces compositions présentent toutefois l'inconvénient de laisser un dépôt indésirable sur les cheveux qui nuit aux propriétés cosmétiques de ces derniers : ainsi, en fin d'opération, les cheveux sont peu brillants, rêches ou collants.

La mise en oeuvre de dérivés siliconés en association avec des résines polymères est connue dans la préparation de compositions cosmétiques pour le maintien de la coiffure. Il a été constaté que ces dérivés siliconés améliorent de façon nette les propriétés de démêlage, de douceur et de brillance des cheveux traités à l'aide de ces compositions, bien que ces propriétés soient inférieures aux propriétés du dérivé de silicone utilisé seul. Cependant, ces dérivés siliconés ne sont pas favorables au pouvoir fixant de ces compositions, assuré par la résine polymère.

En particulier, les silicones oxyalkylénées sont connues pour ne pas conférer de la tenue à la chevelure.

La demande GB 2 173 515 décrit des compositions de teinture pour les cheveux comprenant un colorant direct, un tensioactif siliconé cationique, une silicone ayant une fonction hydroxyle et un véhicule liquide. Ces compositions ne contiennent pas de silicones modifiées par des groupements oxyalkylénés.

Le brevet US 5.188.756 décrit une composition de nettoyage et de conditionnement pour application topique comprenant un émollient, un produit filmogène, un condensat de sucre polyaminé, un tensioactif, un composé cationique tel que le chlorure de gomme de guar hydroxypropyl triméthyl ammonium et de l'eau. L'exemple 3 contient entre autre une gomme de guar cationique et deux silicones, une cyclométhicone et une diméthiconol. Ces deux silicones ne sont pas des silicones modifiées par des groupements oxyalkylénés. D'autre part, cette composition est une composition lavante.

Le document EP-A-0 633 018 décrit un mélange d'une huile de silicone et d'un polydiméthylsiloxane polyoxyéthy- léné polyoxypropylé. L'exemple 1 décrit une composition de shampooing contenant une gomme de guar (JAGUAR HP 60) et une silicone modifiée par des groupements oxyalkylénés (SILBIONE 70646). Cette composition est une composition lavante.

Le document EP-A-0 562 638 décrit dans l'exemple une composition de shampooing conditionneur contenant en particulier une gomme de guar cationique et une silicone modifiée par des groupements oxyalkylénés. Cette composition est une composition lavante.

Le brevet US 5.217.652 décrit une composition de shampooing conditionneur contenant en solution aqueuse un tensioactif anionique, un diméthicone copolyol, le chlorure de gomme de guar hydroxypropyl triméthyl ammonium, un chlorure d'hydroxypropyl bis-isostéaramido propyldiméthyl ammonium, un sel d'aluminium et d'acide minéral et un tensioactif cationique fluoré. Cette composition est une composition lavante.

Aucun de ces document n'évoque le problème de la tenue de la coiffure.

La demande de brevet FR 2 709 954 est un droit national antérieur qui décrit l'association d'un copolymère bloc linéaire polysiloxane-polyoxyalkylène et d'une gomme de guar hydroxypropylée et quaternisée par du chlorure de 2,3- époxypropyl triméthyl ammonium.

Les gommes de guar sont décrites comme étant des agents épaississants. En particulier, elles ont été utilisées pour épaissir des compositions de conditionnement contenant des silicones volatiles dans la demande EP-A-0 035 901.

Or, la demanderesse vient maintenant de découvrir que l'association de gommes de guar avec des silicones oxyalkylénées dans un rapport particulier conduisait de façon inattendue et surprenante à des propriétés particulièrement intéressantes, notamment une amélioration de la tenue de la coiffure dans le temps. La chevelure a plus de volume, les cheveux sont brillants, et présentent un toucher naturel.

Cette découverte est à la base de la présente invention.

La présente invention a donc pour objet de nouvelles compositions cosmétiques non lavantes des matières kératiniques telles que les cheveux, la peau ou les cils, caractérisées en ce qu'elles contiennent, dans un milieu cosmétiquement acceptable non détergent, au moins une gomme de guar et au moins une silicone oxyalkylénée dans un rapport pondéral gomme de guar/silicone inférieur ou égal à 5.

D'autres caractéristiques, aspects, objets et avantages de l'invention apparaîtront encore plus clairement à la lecture de la description et des exemples qui suivent.

Les gommes de guar utilisables selon l'invention peuvent être non ioniques ou cationiques.

Selon l'invention, on peut utiliser les gommes de guar non ioniques chimiquement modifiées ou non modifiées.

Les gommes de guar non ioniques non modifées sont par exemple les produits vendus sous la dénomination VIDOGUM GH 175 par la société UNIPECTINE et sous la dénomination JAGUAR C par la société MEYHALL.

Les gommes de guar non-ioniques modifiées utilisables selon l'invention sont de préférence modifiées par des groupements hydroxyalkyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d<alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.

Les gommes de guar cationiques plus particulièrement utilisables selon l'invention sont des gommes de guar comportant des groupements cationiques trialkylammonium. De préférence, 2 à 30 % et encore plus préférentiellement 5 à 20 % en nombre des fonctions hydroxyle de ces gommes de guar portent des groupements cationiques trialkyammo- nium.

Parmi ces groupements trialkylammonium, on peut tout particulièrement citer les groupements triméthylammonium et triéthylammonium.

Encore plus préférentiellement, ces groupements représentent de 5 à 20 % en poids par rapport au poids total de la gomme de guar modifiée.

Selon l'invention, on utilise de préférence une gomme de guar modifiée par du chlorure de 2,3-époxypropyl triméthylammonium.

Ces gommes de guar modifiées par des groupements cationiques sont des produits déjà connus en eux-mêmes et sont par exemple décrits dans les brevets US 3 589 578 et US 4 031 307. De tels produits sont par ailleurs vendus notamment sous les dénominations commerciales de JAGUAR C13 S, JAGUAR C 15, JAGUAR C 17 par la société MEYHALL.

De préférence, on utilise une gomme de guar non ionique et, parmi ces gommes de guar non ioniques, plus particulièrement les gommes de guar modifiées par des groupement hydroxyalkyles.

Les silicones modifiées par des groupements oxyalkylènes sont par exemple choisies parmi les composés de formules générales (I), (II), (III) et (IV): 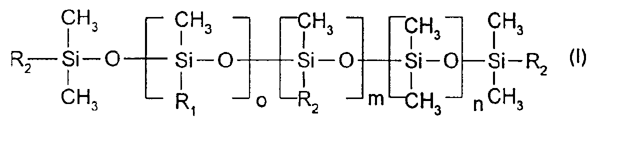 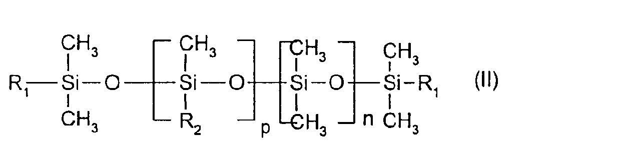 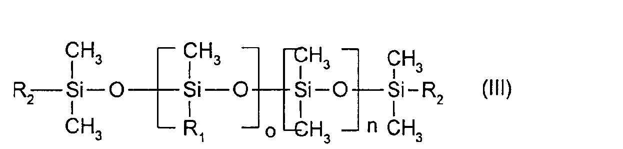 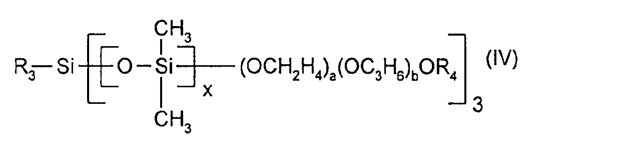 formule dans lesquelles :
- R₁ représente un radical alkyle en C₁-C₃₀ ou phényle.
- R₂ représente (CH₂)_{c}(-C₂H₄O)ₐ(-C₃H₆O)_{b}-R₅,
- R₃, R4, identiques ou différents, désignent un radical alkyle en C₁-C₁₂
- R₅ est choisi parmi un atome d'hydrogène, un radical alkyle de 1 à 12 atomes de carbone, un radical alcoxy de 1 à 6 atomes de carbone, un radical hydroxyle, - S0₃M, -OCOR₆, aminoalcoxy en C₁-C₆ éventuellement substitué sur l'amine, aminoacyle en C₁-C₆ éventuellement substitué sur l'amine, -NHCH₂CH₂COOM, N(CH₂CH₂COOM)₂,aminoalkyle éventuellement substitué sur l'amine et sur la chaîne alkyle, carboxyacyle en C₁-C₃₀, un groupement phosphono éventuellement substitué par un ou deux radicaux aminoalkyle substitué, - CO(CH₂)_{d}COOM, - OCOCHR₇(CH₂)_{d}COOM, -NHCO(CH₂)_{d}OH, NH₃Y
- M désigne un atome d'hydrogène, Na, K, Li, NH₄ ou une amine organique,
R₆ désigne un radical alkyle en C₁-C₃₀,
- R₇ désigne hydrogène ou SO₃M,
- d varie de 1 à 10,
m varie de 0 à 20,
- n varie de 1 à 100,
o varie de 0 à 20,
- p varie de 1 à 20,
- a varie de 0 à 50,
b varie de 0 à 50,
- a + b est supérieur ou égal à 1,
- c varie de 0 à 4,
x varie de 1 à 100,
- Y représente un anion minéral ou organique monovalent.

De préférence, on utilise les silicones répondant à la formule générale (I) ou (II), et en particulier dans lesquelles R5 désigne un atome d'hydrogène.

De façon préférentielle, c est égal 2 ou 3.

De telles silicones sont par exemple vendues par la société GOLDSCHMIDT sous les dénominations commerciales ABIL WE 09, ABIL EM 90, ABIL B8852, ABIL B8851, ABIL B 8843, ABIL B8842, par la société DOW CORNING sous les dénominations FLUID DC 190, DOW CORNING 193, DC 3225 C, Q2-5220, Q2-5354, Q2-5200, par la société RHONE POULENC sous les dénominations SILBIONE HUILE 70646, RHODORSIL HUILE 10634, par la société GENERAL ELECTRIC sous les dénominations SF1066, SF1188, par la société SWS SILICONES sous la dénomination SILICONE COPOLYMER F 754, par la société AMERCHOL sous la dénomination SILSOFT BEAUTY AID SL, par la société SHINETSU sous la dénomination KF 351, par la société WACKER sous la dénomination BELSIL DMC 6038, par la société SILTECH sous les dénominations SILWAX WD-C, SILWAX WD-B, SILWAX WD-IS, SILWAX WS-L, SILWAX DCA 100, SILTECH AMINE 65, par la société FANNING CORPORATION sous les dénominations FANCORSIL SLA, FANCORSIL LIM1.

La concentration en gomme de guar peut varier entre 0,01 % et 5 % en poids environ par rapport au poids total de la composition, et de préférence entre 0,1 et 2,5 % environ.

La concentration en silicone oxyalkylénée peut varier entre 0,01 et 5 % en poids environ par rapport au poids total de la composition, et de préférence entre 0,1 et 2,5 % environ.

Le rapport pondéral gomme de guar/silicone oxyalkylénée est de préférence compris entre 0,1 et 4.

Le milieu cosmétiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Le pH des compositions selon l'invention est généralement compris entre 2 et 9, et en particulier entre 3 et 8. Il peut être ajusté à la valeur choisie au moyen d'agents alcalinisants ou acidifiants habituellement utilisés en cosmétique pour ce type d'application.

Les compositions selon l'invention peuvent encore contenir, des agents épaississants, des agents tensioactifs, des agents conservateurs, des séquestrants, des adoucissants, des parfums, des colorants, des agents modificateurs de viscosité, des agents modificateurs de mousse, des agents moussants, des stabilisateurs de mousse, des agents nacrants, des agents hydratants, des agents antipelliculaires, des agents antiséborrhéiques, des filtres solaires, des protéines, des vitamines, des plastifiants, des hydroxyacides, des électrolytes, des agents propulseurs et des parfums.

Les compositions selon l'invention peuvent également contenir des agents conditionneurs. Ceux-ci peuvent alors être choisis parmi les huiles et les cires naturelles ou synthétiques, les alcools gras, les esters d'alcools polyhydriques, les glycérides, les huiles, les gommes et résines de silicone ou les mélanges de ces différents composes.

L'invention a encore pour objet un procédé de traitement cosmétique non avant des matières kératiniques, telles que la peau, les cheveux ou les cils, caractérisé en ce qu'il consiste à appliquer sur les matières kératiniques une composition cosmétique telle que définie précédemment, à éventuellement rincer, après un éventuel temps de pose.

Ainsi, ce procédé selon l'invention permet le maintien de la coiffure ou des cils, le traitement, le soin de la peau, des cheveux ou de toute autre matière kératinique.

Les compositions cosmétiques selon l'invention peuvent se présenter sous forme de gel, de lait, de crème, de gel-crème, de spray, de lotion plus ou moins épaissie ou de mousse et être utilisées par exemple pour la peau, les cheveux, les cils ou les sourcils.

Pour les cheveux, elles sont plus particulièrement des compositions à rincer ou non, à appliquer avant ou après un shampooing, une coloration, une décoloration, une permanente ou un défrisage.

Les compositions peuvent être également des lotions de mise en plis, des lotions pour le brushing, des compositions de fixation et de coiffage.

Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote ou l'air comprimé.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

### EXEMPLE 1

On a préparé un gel (1), conforme à l'invention, de composition suivante :

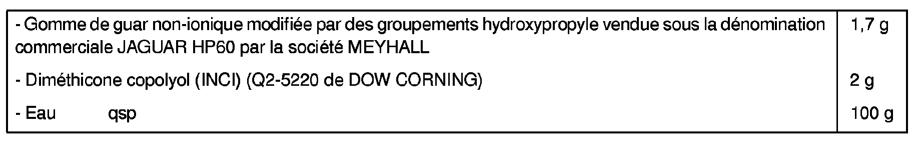

On a préparé un gel (2), selon l'invention, de composition suivante :

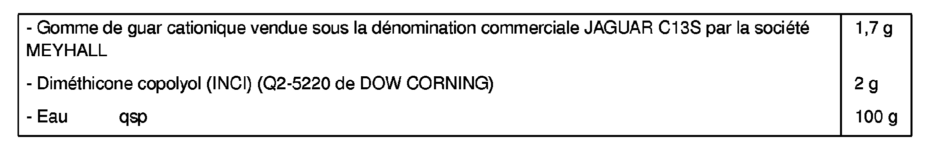

On a préparé un gel (3), comparatif, de composition suivante :

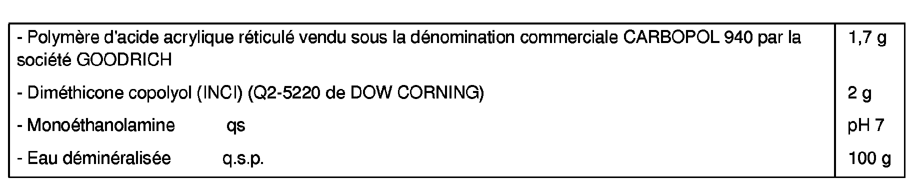

On a préparé un gel (4), comparatif, de composition suivante :

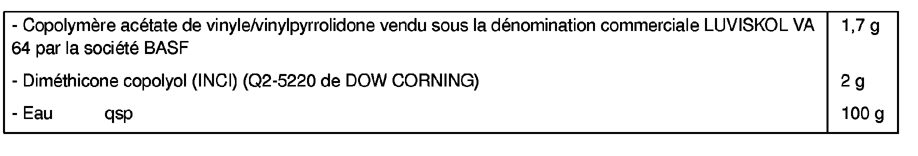

Sur des mèches de cheveux lavés et essorés, on a appliqué 0,5 g de gel par g de cheveux. On a ensuite procédé à un brushing.

On a ensuite demandé à un panel de 8 juges d'évaluer le plixant (durcissement de la fibre) et le toucher des cheveux.

### Notation :

Plixant : 0 (pas de plixant) à 5 (fixation très forte)
Toucher : 0 (très mauvais) à 5 (excellent)

Après 5 heures à température et humidité ambiante, les mêmes juges ont évalué la tenue de la coiffure.

### Notation :

Tenue : 0 (pas de tenue) à 5 (tenue parfaite)

Les résultats (moyenne des notes) sont rassemblés dans le tableau ci-dessous :

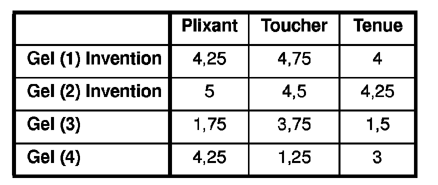

Seuls les gels 1 et 2 selon l'invention présentent de très bonnes propriétés de plixant, de toucher et de tenue.

On a également préparé un gel (5), comparatif, de composition suivante :

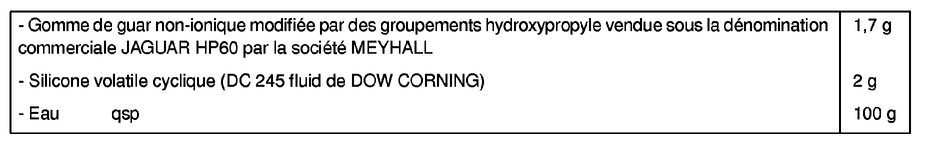

On a également préparé un gel (6), comparatif, de composition suivante :

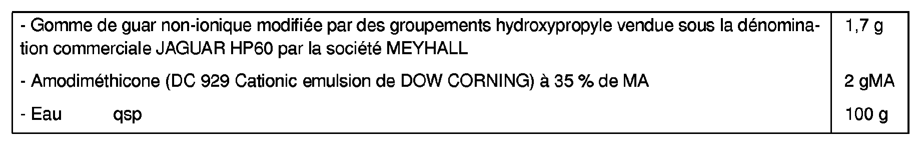

Sur des mèches de cheveux lavés et essorés, on a appliqué 0,5 g de gel par g de cheveux. On a ensuite procédé à un brushing.

On a ensuite demandé à un panel de 8 juges d'évaluer le plixant, le toucher et la tenue de la coiffure.

Les résultats (moyenne des notes) sont rassemblés dans le tableau ci-dessous :

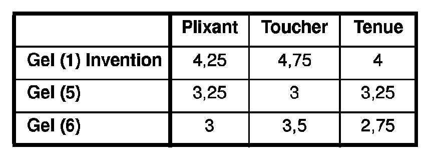

Le gel (1) selon l'invention présente de meilleures propriétés de plixant, de toucher et de tenue que les compositions contenant des silicones déjà utilisées dans les compositions de l'art antérieur.

### EXEMPLE 2

On a préparé un gel de composition suivante :

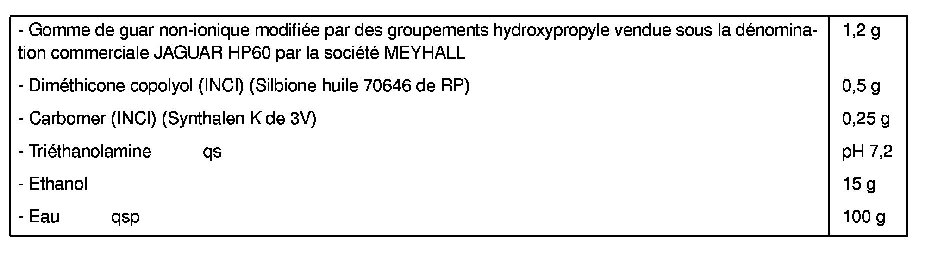

Ce gel est appliqué sur cheveux mouillés (environ 5 g par tête) puis on procède à un brushing.

Les cheveux sont disciplinés, brillants et présentent un toucher naturel.
La tenue de la coiffure obtenue est très bonne.

On peut également appliquer ce gel sur cheveux secs. En déposant le gel, on met en forme la chevelure avec les mains.
La tenue de la coiffure obtenue est très bonne.

### EXEMPLE 3

On a préparé un gel de composition suivante :

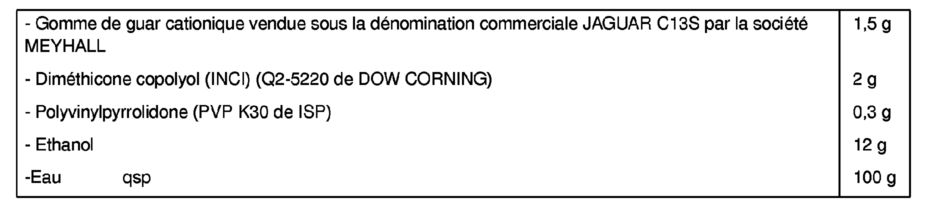

Ce gel est appliqué sur cheveux lavés et humides (environ 10 g par tête), on décole les racines puis on procède à séchage au casque pendant environ 30 minutes. Les cheveux sont ensuite brossés.
La coiffure est gonflante, les cheveux présentent un toucher naturel.
La tenue de la coiffure obtenue est très bonne.

### EXEMPLE 4

On a préparé une mousse de coiffage de composition suivante :

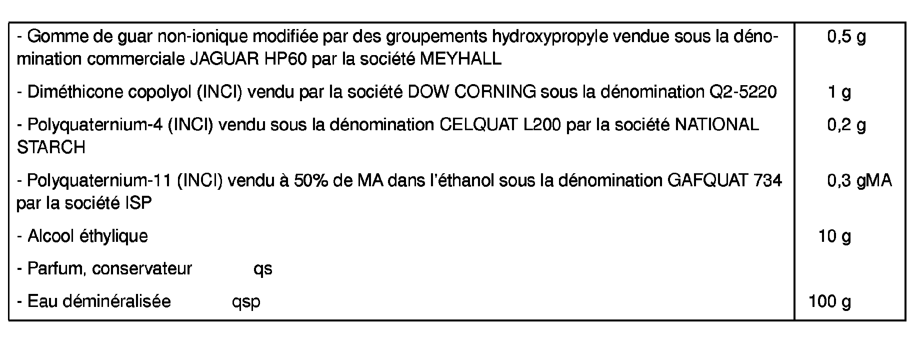

### Conditionnement en aérosol :

90g de la composition ci-dessus sont conditionnés dans un récipient aérosol en présence de 10g d'un mélange ternaire de n-butane, isobutane et propane, (23/55/22), vendu sous la dénomination de "AEROGAZ 3,2 N par la société ELF AQUITAINE

Cette mousse présente une texture crémeuse très agréable.
Cette mousse a été appliquée sur les cheveux mouillés, elle se répartit facilement sur l'ensemble de la chevelure et facilite le démêlage des cheveux.
On procède à un brushing, la coiffure obtenue est gonflante, les cheveux sont brillants, sans résidus et le toucher est naturel.
La coiffure présente une bonne tenue dans le temps.

## Revendications (Revendications pour l'(les) Etat(s) contractant(s) suivant(s) : AT, BE, CH, DE, ES, GB, IT, LI, NL, SE)

1. Composition cosmétique non lavante, caractérisée par le fait qu'elle contient, dans un milieu cosmétiquement acceptable, au moins une gomme de guar et au moins une silicone modifiée par des groupements oxyalkylénés dans un rapport pondéral gomme de guar/silicone inférieur ou égal à 5.

2. Composition selon la revendication 1, caractérisée par le fait la gomme de guar est choisie parmi les gommes de guar non ioniques ou cationiques.

3. Composition selon la revendication 2, caractérisée par le fait la gomme de guar est choisie parmi les gommes de guar non ioniques.

4. Composition selon la revendication 3, caractérisée par le fait que la gomme de guar non-ionique est modifiée par des groupements hydroxyalkyle en C↑ -Cε.

5. Composition selon la revendication 4, caractérisée par le fait que les groupements hydroxyalkyle sont choisis parmi les groupements hydroxyméthyle, hydroxyéthyle, hydroxypropyle et hydroxybutyle.

6. Composition selon l'une quelconque des revendications 3 à 5, caractérisée par le fait que la gomme de guar non-ionique présente un taux d'hydroxyalkylation variant entre 0,4 et 1,2.

7. Composition selon la revendication 1 ou 2, caractérisée par le fait que la gomme de guar cationique contient des groupements trialkylammonium.

8. Composition selon la revendication 7, caractérisée par le fait que les groupements trialkylammonium sont choisis parmi les groupements triméthylammonium et triéthylammonium.

9. Composition selon l'une quelconque des revendications 7 ou 8, caractérisée par le fait que les groupements trialkylammonium représentent moins de 30 % en poids par rapport au poids total de la gomme de guar modifiée.

10. Composition selon la revendication 9, caractérisée par le fait que les groupements trialkylammonium représentent de 5 à 20 % en poids par rapport au poids total de la gomme de guar modifiée.

11. Composition selon l'une quelconque des revendications 7 à 10, caractérisée par le fait que la gomme de guar est modifiée par du chlorure de 2,3-époxypropyl triméthylammonium.

12. Composition selon l'une quelconque des revendications précédentes, caractérisée par le fait que les silicones modifiées par des groupements oxyalkylènes sont choisies parmi les composés de formules générales (I), (II), (III) et (IV): 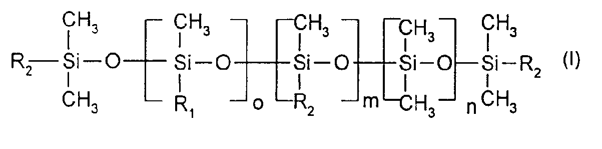 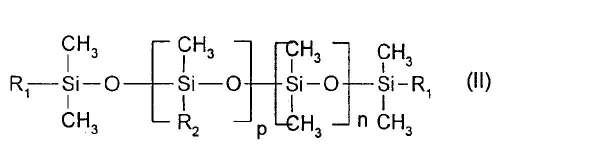 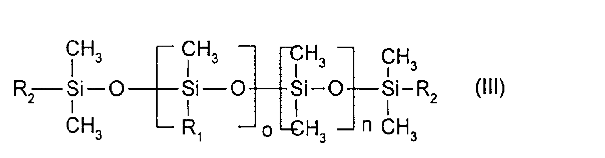 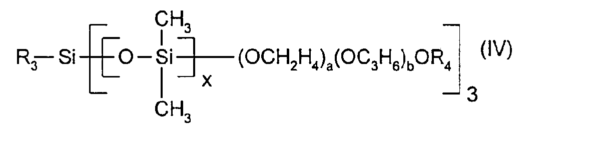 formule dans lesquelles :
- R₁ représente un radical alkyle en C₁-C₃₀ ou phényle.
- R₂ représente (CH₂)_{c}(-C₂H₄O)ₐ(-C₃H₆O)_{b}-R₅
R₃, R₄ désignent un radical alkyle en C₁-C₁₂
- R₅ est choisi parmi un atome d'hydrogène, un radical alkyle de 1 à 12 atomes de carbone, un radical alcoxy de 1 à 6 atomes de carbone, un radical hydroxyle, - SO₃M, -OCOR₆, aminoalcoxy en C₁-C₆ éventuellement substitué sur l'amine, aminoacyle en C₁-C₆ éventuellement substitué sur l'amine, -NHCH₂CH₂COOM, N(CH₂CH₂COOM)₂,aminoalkyle éventuellement substitué sur l'amine et sur la chaîne alkyle, carboxyacyle en C₁-C₃₀, un groupement phosphono éventuellement substitué par un ou deux radicaux aminoalkyle substitué, -CO(CH₂)_{d}COOM, - OCOCHR₇(CH₂)_{d}COOM, -NHCO(CH₂)_{d}OH, NH₃Y.
- M désigne un atome d'hydrogène, Na, K, Li, NH₄ ou une amine organique,
R₆ désigne un radical alkyle en C₁-C₃₀,
- R₇ désigne hydrogène ou SO₃M,
- d varie de 1 à 10,
m varie de 0 à 20,
- n varie de 1 à 100,
- o varie de 0 à 20,
- p varie de 1 à 20,
- a varie de 0 à 50,
- b varie de 0 à 50,
a + b est supérieur ou égal à 1,
- c varie de 0 à 4,
- x varie de 1 à 100,
- Y est un anion minéral ou organique monovalent.

13. Composition selon l'une quelconque des revendications 1 à 12, caractérisée par le fait que les silicones répondent à la formule générale (I) ou (II).

14. Composition selon la revendication 13, caractérisée par le fait que R5 désigne un atome d'hydrogène.

15. Composition selon l'une quelconque des revendications 1 à 14, caractérisée par le fait que les gommes de guar sont présents dans des proportions comprises entre 0,01 % et 5 % en poids environ par rapport au poids total de la composition, et de préférence entre 0,1 et 2,5 %.

16. Composition selon l'une quelconque des revendications 1 à 15, caractérisée par le fait que les silicones oxyalkylénées sont présentes dans des proportions comprises entre 0,01 et 5 % en poids environ par rapport au poids total de la composition, et de préférence entre 0,1 et 2,5 %.

17. Composition selon l'une quelconque des revendications 1 à 16, caractérisée par le fait que le milieu cosmétiquement acceptable est constitué par de l'eau, un mélange d'eau et de solvants cosmétiquement acceptables choisis parmi les monoalcools, les polyalcools, les éthers de glycol ou les esters d'acides gras, utilisés seuls ou en mélange.

18. Composition selon l'une quelconque des revendications 1 à 17, caractérisée par le fait qu'elle contient également des agents tensio-actifs, des agents épaississants, des agents conservateurs, des séquestrants, des adoucissants, des parfums, des colorants, des agents modificateurs de viscosité, des agents modificateurs de mousse, des agents moussants, des stabilisateurs de mousse, des agents nacrants, des agents hydratants, des agents antipelliculaires, des agents antiséborrhéiques, des filtres solaires, des protéines, des vitamines, des plastifiants, des hydroxyacides, des électrolytes, des agents propulseurs, des parfums, et des agents conditionneurs.

19. Composition selon l'une quelconque des revendications 1 à 18, caractérisée par le fait qu'elle se présente sous forme de gel, de lait, de crème, de gel-crème, de spray, de lotion plus ou moins épaisse ou de mousse.

20. Procédé de traitement cosmétique non lavant des matières kératiniques, caractérisé par le fait que l'on applique sur des matières kératiniques une composition telle que définie à l'une quelconque des revendications 1 à 19, puis que l'on procède éventuellement à un rinçage, après un éventuel temps de pose.

21. Procédé selon la revendication 20, caractérisé par le fait que les matières kératiniques sont choisies parmi les cheveux et les cils.

## Revendications (Revendications pour l'(les) Etat(s) contractant(s) suivant(s) : FR)

1. Composition cosmétique non lavante, caractérisée par le fait qu'elle contient, dans un milieu cosmétiquement acceptable, au moins une gomme de guar et au moins une silicone modifiée par des groupements oxyalkylénés dans un rapport pondéral gomme de guar/silicone inférieur ou égal à 5, à l'exception de l'association d'un copolymère bloc linéaire polysiloxane-polyoxyalkylène et d'une gomme de guar hydroxypropylée et quaternisée par du chlorure de 2,3-époxypropyl triméthyl ammonium.

2. Composition selon la revendication 1, caractérisée par le fait la gomme de guar est choisie parmi les gommes de guar non ioniques ou cationiques.

3. Composition selon la revendication 2, caractérisée par le fait la gomme de guar est choisie parmi les gommes de guar non ioniques.

4. Composition selon la revendication 3, caractérisée par le fait que la gomme de guar non-ionique est modifiée par des groupements hydroxyalkyle en C₁-C₆.

5. Composition selon la revendication 4, caractérisée par le fait que les groupements hydroxyalkyle sont choisis parmi les groupements hydroxyméthyle, hydroxyéthyle, hydroxypropyle et hydroxybutyle.

6. Composition selon l'une quelconque des revendications 3 à 5, caractérisée par le fait que la gomme de guar non-ionique présente un taux d'hydroxyalkylation variant entre 0,4 et 1,2.

7. Composition selon la revendication 1 ou 2, caractérisée par le fait que la gomme de guar cationique contient des groupements trialkylammonium.

8. Composition selon la revendication 7, caractérisée par le fait que les groupements trialkylammonium sont choisis parmi les groupements triméthylammonium et triéthylammonium.

9. Composition selon l'une quelconque des revendications 7 ou 8, caractérisée par le fait que les groupements trialkylammonium représentent moins de 30 % en poids par rapport au poids total de la gomme de guar modifiée.

10. Composition selon la revendication 9, caractérisée par le fait que les groupements trialkylammonium représentent de 5 à 20 % en poids par rapport au poids total de la gomme de guar modifiée.

11. Composition selon l'une quelconque des revendications 7 à 10, caractérisée par le fait que la gomme de guar est modifiée par du chlorure de 2,3-époxypropyl triméthylammonium.

12. Composition selon l'une quelconque des revendications précédentes, caractérisée par le fait que les silicones modifiées par des groupements oxyalkylènes sont choisies parmi les composés de formules générales (1), (II), (III) et (IV): 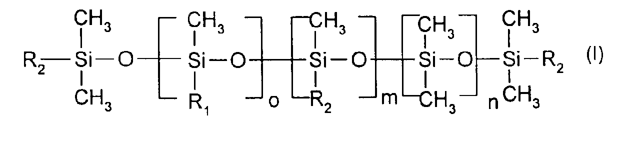 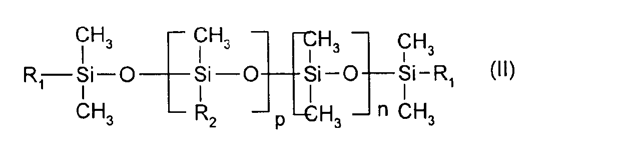 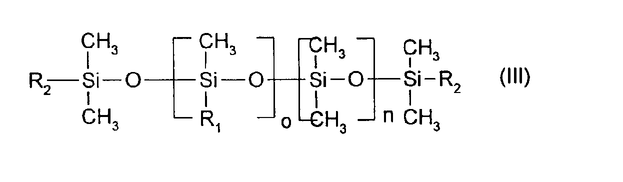 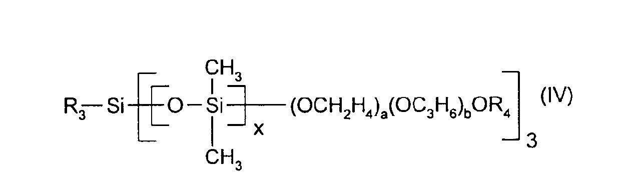 formules dans lesquelles :
- R₁ représente un radical alkyle en C₁-C₃₀ ou phényle.
- R₂ représente (CH₂)_{c}(-C₂H₄O)ₐ(-C₃H₆O)_{b}-R₅,
- R₃, R₄ désignent un radical alkyle en C₁-C₁₂
R₅ est choisi parmi un atome d'hydrogène, un radical alkyle de 1 à 12 atomes de carbone, un radical alcoxy de 1 à 6 atomes de carbone, un radical hydroxyle, - S0₃M, -OCOR₆, aminoalcoxy en Ci-C₆ éventuellement substitué sur l'amine, aminoacyle en Ci-C₆ éventuellement substitué sur l'amine, -NHCH₂CH₂COOM, N(CH₂CH₂COOM)₂, aminoalkyle éventuellement substitué sur l'amine et sur la chaîne alkyle, carboxyacyle en C₁-C₃₀, un groupement phosphono éventuellement substitué par un ou deux radicaux aminoalkyle substitué, -CO(CH₂)_{d}COOM, - OCOCHR₇(CH₂)_{d}COOM, -NHCO(CH₂)_{d}OH, NH3Y.
- M désigne un atome d'hydrogène, Na, K, Li, NH₄ ou une amine organique,
- R₆ désigne un radical alkyle en C₁-C₃₀,
R₇ désigne hydrogène ou S0₃M,
- d varie de 1 à 10,
- m varie de 0 à 20,
n varie de 1 à 100,
- o varie de 0 à 20,
- p varie de 1 à 20,
a varie de 0 à 50,
- b varie de 0 à 50,
a + b est supérieur ou égal à 1,
- c varie de 0 à 4
- x varie de 1 à 100,
Y est un anion minéral ou organique monovalent.

13. Composition selon l'une quelconque des revendications 1 à 12, caractérisée par le fait que les silicones répondent à la formule générale (I) ou (II).

14. Composition selon la revendication 13, caractérisée par le fait que R5 désigne un atome d'hydrogène.

15. Composition selon l'une quelconque des revendications 1 à 14, caractérisée par le fait que les gommes de guar sont présents dans des proportions comprises entre 0,01 % et 5 % en poids environ par rapport au poids total de la composition, et de préférence entre 0,1 et 2,5 %.

16. Composition selon l'une quelconque des revendications 1 à 15, caractérisée par le fait que les silicones oxyalkylénées sont présentes dans des proportions comprises entre 0,01 et 5 % en poids environ par rapport au poids total de la composition, et de préférence entre 0,1 et 2,5 %.

17. Composition selon l'une quelconque des revendications 1 à 16, caractérisée par le fait que le milieu cosmétiquement acceptable est constitué par de l'eau, un mélange d'eau et de solvants cosmétiquement acceptables choisis parmi les monoalcools, les polyalcools, les éthers de glycol ou les esters d'acides gras, utilisés seuls ou en mélange.

18. Composition selon l'une quelconque des revendications 1 à 17, caractérisée par le fait qu'elle contient également des agents tensio-actifs, des agents épaississants, des agents conservateurs, des séquestrants, des adoucissants, des parfums, des colorants, des agents modificateurs de viscosité, des agents modificateurs de mousse, des agents moussants, des stabilisateurs de mousse, des agents nacrants, des agents hydratants, des agents antipelliculaires, des agents antiséborrhéiques, des filtres solaires, des protéines, des vitamines, des plastifiants, des hydroxyacides, des électrolytes, des agents propulseurs, des parfums, et des agents conditionneurs.

19. Composition selon l'une quelconque des revendications 1 à 18, caractérisée par le fait qu'elle se présente sous forme de gel, de lait, de crème, de gel-crème, de spray, de lotion plus ou moins épaisse ou de mousse.

20. Procédé de traitement cosmétique non lavant des matières kératiniques, caractérisé par le fait que l'on applique sur des matières kératiniques une composition telle que définie à l'une quelconque des revendications 1 à 19, puis que l'on procède éventuellement à un rinçage, après un éventuel temps de pose.

21. Procédé selon la revendication 20, caractérisé par le fait que les matières kératiniques sont choisies parmi les cheveux et les cils.

## Claims (Claims for the following Contracting State(s) : AT, BE, CH, DE, ES, GB, IT, LI, NL, SE)

1. Non-washing cosmetic composition, characterized in that it contains, in a cosmetically acceptable medium, at least one guar gum and at least one silicone modified with oxyalkylenated groups in a guar gum/silicone weight ratio of less than or equal to 5.

2. Composition according to Claim 1, characterized in that the guar gum is chosen from nonionic or cationic guar gums.

3. Composition according to Claim 2, characterized in that the guar gum is chosen from nonionic guar gums.

4. Composition according to Claim 3, characterized in that the nonionic guar gum is modified with C₁-C₆ hydroxyalkyl groups.

5. Composition according to Claim 4, characterized in that the hydroxyalkyl groups are chosen from hydroxymethyl, hydroxyethyl, hydroxypropyl and hydroxybutyl groups.

6. Composition according to any one of Claims 3 to 5, characterized in that the nonionic guar gum has a degree of hydroxyalkylation ranging between 0.4 and 1.2.

7. Composition according to Claim 1 or 2, characterized in that the cationic guar gum contains trialkylammonium groups.

8. Composition according to Claim 7, characterized in that the trialkylammonium groups are chosen from trimethylammonium and triethylammonium groups.

9. Composition according to either of Claims 7 and 8, characterized in that the trialkylammonium groups represent less than 30 % by weight relative to the total weight of the modified guar gum.

10. Composition according to Claim 9, characterized in that the trialkylammonium groups represent from 5 to 20 % by weight relative to the total weight of the modified guar gum.

11. Composition according to any one of Claims 7 to 10, characterized in that the guar gum is modified with 2,3-epoxypropyltrimethylammonium chloride.

12. Composition according to any one of the preceding claims, characterized in that the silicones modified with oxyalkylene groups are chosen from the compounds of general formulae (I), (II), (III) and (IV): 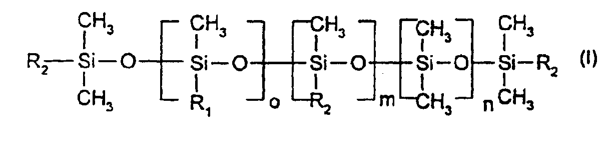 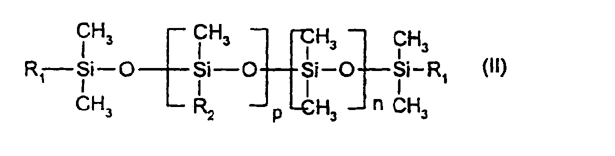 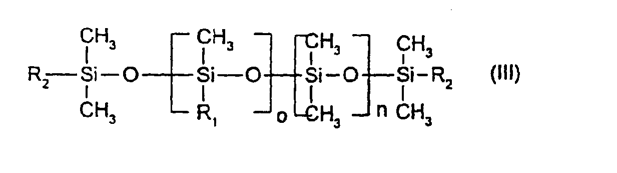 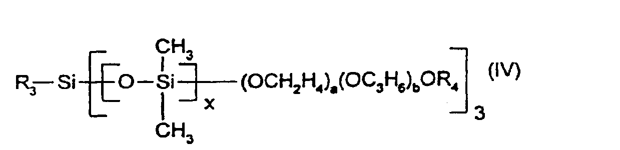 in which formulae:
- R₁ represents a phenyl or C₁-C₃₀ alkyl radical,
- R₂ represents (CH₂)_{c}(-C₂H₄O)ₐ(-C₃H₆O)_{b}-R₅,
- R₃ and R₄ denote a C₁-C₁₂ alkyl radical,
R₅ is chosen from a hydrogen atom, an alkyl radical of 1 to 12 carbon atoms, an alkoxy radical of 1 to 6 carbon atoms, a hydroxyl radical, -SO₃M, -OCOR₆, C₁-C₆ aminoalkoxy optionally substituted on the amine, C₁-C₆ aminoacyl optionally substituted on the amine, -NHCH₂CH₂COOM, N(CH₂CH₂COOM)₂, aminoalkyl optionally substituted on the amine and on the alkyl chain, C₁-C₃₀ carboxyacyl, a phosphono group optionally substituted with one or two substituted aminoalkyl radicals, -CO(CH₂)_{d}COOM, -OCOCHR₇(CH₂)_{d}COOM, - NHCO(CH₂)_{d}OH and NH₃Y,
M denotes a hydrogen atom, Na, K, Li, NH₄ or an organic amine,
- R₆ denotes a C₁-C₃₀ alkyl radical,
- R₇ denotes hydrogen or SO₃M,
d ranges from 1 to 10,
- m ranges from 0 to 20,
n ranges from 1 to 100,
- o ranges from 0 to 20,
- p ranges from 1 to 20,
a ranges from 0 to 50,
- b ranges from 0 to 50,
- a + b is greater than or equal to 1,
c ranges from 0 to 4,
- x ranges from 1 to 100,
- Y is a monovalent inorganic or organic anion.

13. Composition according to any one of Claims 1 to 12, characterized in that the silicones correspond to the general formula (I) or (II).

14. Composition according to Claim 13, characterized in that R₅ denotes a hydrogen atom.

15. Composition according to any one of Claims 1 to 14, characterized in that the guar gums are present in proportions of between 0.01 % and 5 % by weight approximately relative to the total weight of the composition, and preferably of between 0.1 and 2.5 %.

16. Composition according to any one of Claims 1 to 15, characterized in that the oxyalkylenated silicones are present in proportions of between 0.01 and 5 % by weight approximately relative to the total weight of the composition, and preferably of between 0.1 and 2.5 %.

17. Composition according to any one of Claims 1 to 16, characterized in that the cosmetically acceptable medium consists of water or a mixture of water and cosmetically acceptable solvents chosen from monoalcohols, polyalcohols, glycol ethers or fatty acid esters, which are used alone or as a mixture.

18. Composition according to any one of Claims 1 to 17, characterized in that it also contains surfactants, thickeners, preserving agents, sequestering agents, softeners, fragrances, dyes, viscosity modifiers, foam modifiers, foaming agents, foam stabilizers, pearling agents, moisturizing agents, antidandruff agents, antiseborrhoeic agents, sunscreens, proteins, vitamins, plasticizers, hydroxy acids, electrolytes, propellants, fragrances and conditioners.

19. Composition according to any one of Claims 1 to 18, characterized in that it is in the form of a gel, a milk, a cream, a cream-gel, a spray, a more or less thickened lotion or a mousse.

20. Process for the non-washing cosmetic treatment of keratinous material, characterized in that a composition as defined in any one of Claims 1 to 19 is applied to keratinous material, optionally followed by rinsing, after an optional period of exposure of the keratinous material to the composition.

21. Process according to Claim 20, characterized in that the keratinous material is chosen from the hair and the eyelashes.

## Claims (Claims for the following Contracting State(s) : FR)

1. Non-washing cosmetic composition, characterized in that it contains, in a cosmetically acceptable medium, at least one guar gum and at least one silicone modified with oxyalkylenated groups in a guar gum/silicone weight ratio of less than or equal to 5, with the exception of a linear block copolymer of polysiloxane-polyoxyalkylene and of a hydroxypropyl guar gum quaternized with 2,3-epoxypropyltrimethylammonium choride.

2. Composition according to Claim 1, characterized in that the guar gum is chosen from nonionic or cationic guar gums.

3. Composition according to Claim 2, characterized in that the guar gum is chosen from nonionic guar gums.

4. Composition according to Claim 3, characterized in that the nonionic guar gum is modified with C1-C6 hydroxyalkyl groups.

5. Composition according to Claim 4, characterized in that the hydroxyalkyl groups are chosen from hydroxymethyl, hydroxyethyl, hydroxypropyl and hydroxybutyl groups.

6. Composition according to any one of Claims 3 to 5, characterized in that the nonionic guar gum has a degree of hydroxyalkylation ranging between 0.4 and 1.2.

7. Composition according to Claim 1 or 2, characterized in that the cationic guar gum contains trialkylammonium groups.

8. Composition according to Claim 7, characterized in that the trialkylammonium groups are chosen from trimethylammonium and triethylammonium groups.

9. Composition according to either of Claims 7 and 8, characterized in that the trialkylammonium groups represent less than 30 % by weight relative to the total weight of the modified guar gum.

10. Composition according to Claim 9, characterized in that the trialkylammonium groups represent from 5 to 20 % by weight relative to the total weight of the modified guar gum.

11. Composition according to any one of Claims 7 to 10, characterized in that the guar gum is modified with 2,3-epoxypropyltrimethylammonium chloride.

12. Composition according to any one of the preceding claims, characterized in that the silicones modified with oxyalkylene groups are chosen from the compounds of general formulae (I), (II), (III) and (IV): 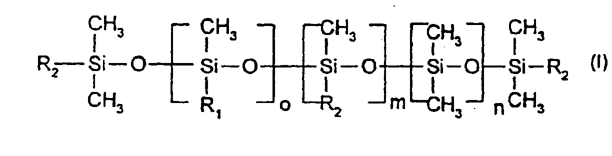 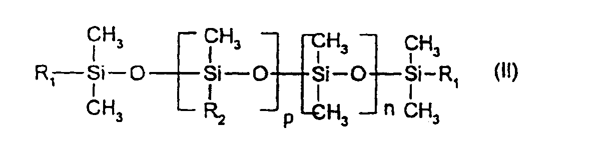 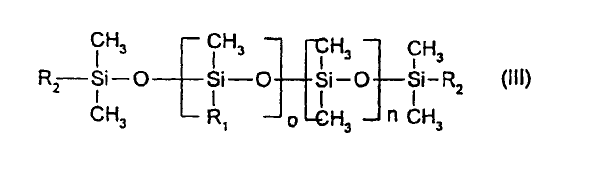 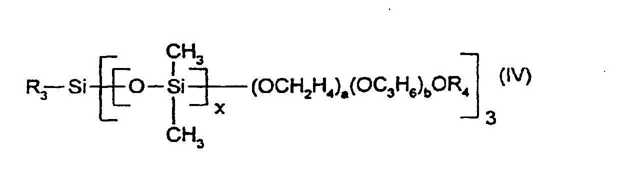 in which formulae:
- R₁ represents a phenyl or C₁-C₃₀ alkyl radical,
- R₂ represents (CH₂)_{c}(-C₂H₄O)ₐ(-C₃H₆O)_{b}-R₅,
- R₃ and R₄ denote a C₁-C₁₂ alkyl radical,
- R₅ is chosen from a hydrogen atom, an alkyl radical of 1 to 12 carbon atoms, an alkoxy radical of 1 to 6 carbon atoms, a hydroxyl radical, -SO₃M, -OCOR₆, C₁-C₆ aminoalkoxy optionally substituted on the amine, C₁-C₆ aminoacyl optionally substituted on the amine, -NHCH₂CH₂COOM, N(CH₂CH₂COOM)₂, aminoalkyl optionally substituted on the amine and on the alkyl chain, C₁-C₃₀ carboxyacyl, a phosphono group optionally substituted with one or two substituted aminoalkyl radicals, -CO(CH₂)_{d}COOM, -OCOCHR₇(CH₂)_{d}COOM, - NHCO(CH₂)_{d}OH and NH₃Y,
M denotes a hydrogen atom, Na, K, Li, NH₄ or an organic amine,
- R₆ denotes a C₁-C₃₀ alkyl radical,
- R₇ denotes hydrogen or SO₃M,
d ranges from 1 to 10,
- m ranges from 0 to 20,
- n ranges from 1 to 100,
o ranges from 0 to 20,
- p ranges from 1 to 20,
- a ranges from 0 to 50,
- b ranges from 0 to 50,
- a + b is greater than or equal to 1,
- c ranges from 0 to 4,
x ranges from 1 to 100,
- Y is a monovalent inorganic or organic anion.

13. Composition according to any one of Claims 1 to 12, characterized in that the silicones correspond to the general formula (I) or (II).

14. Composition according to Claim 13, characterized in that R₅ denotes a hydrogen atom.

15. Composition according to any one of Claims 1 to 14, characterized in that the guar gums are present in proportions of between 0.01 % and 5 % by weight approximately relative to the total weight of the composition, and preferably of between 0.1 and 2.5 %.

16. Composition according to any one of Claims 1 to 15, characterized in that the oxyalkylenated silicones are present in proportions of between 0.01 and 5 % by weight approximately relative to the total weight of the composition, and preferably of between 0.1 and 2.5 %.

17. Composition according to any one of Claims 1 to 16, characterized in that the cosmetically acceptable medium consists of water or a mixture of water and cosmetically acceptable solvents chosen from monoalcohols, polyalcohols, glycol ethers or fatty acid esters, which are used alone or as a mixture.

18. Composition according to any one of Claims 1 to 17, characterized in that it also contains surfactants, thickeners, preserving agents, sequestering agents, softeners, fragrances, dyes, viscosity modifiers, foam modifiers, foaming agents, foam stabilizers, pearling agents, moisturizing agents, antidandruff agents, antiseborrhoeic agents, sunscreens, proteins, vitamins, plasticizers, hydroxy acids, electrolytes, propellants, fragrances and conditioners.

19. Composition according to any one of Claims 1 to 18, characterized in that it is in the form of a gel, a milk, a cream, a cream-gel, a spray, a more or less thickened lotion or a mousse.

20. Process for the non-washing cosmetic treatment of keratinous material, characterized in that a composition as defined in any one of Claims 1 to 19 is applied to keratinous material, optionally followed by rinsing, after an optional period of exposure of the keratinous material to the composition.

21. Process according to Claim 20, characterized in that the keratinous material is chosen from the hair and the eyelashes.

## Patentansprüche (Patentansprüche für folgende(n) Vertragsstaat(en) : AT, BE, CH, DE, ES, GB, IT, LI, NL, SE)

1. Nicht reinigende kosmetische Zusammensetzung, dadurch gekennzeichnet, daß sie in einem kosmetisch akzeptablen Medium mindestens ein Guargummi und mindestens ein mit Oxyalkylengruppen modifiziertes Silicon in einem Gewichtsverhältnis Guargummi/Silicon kleiner oder gleich 5 enthält.

2. Zusammensetzung nach Anspruch 1, dadurch gekennzeichnet, daß das Guargummi unter nichtionischen oder kationischen Guargummen ausgewählt ist.

3. Zusammensetzung nach Anspruch 2, dadurch gekennzeichnet, daß das Guargummi unter nichtionischen Guargummen ausgewählt ist.

4. Zusammensetzung nach Anspruch 3, dadurch gekennzeichnet, daß das nichtionische Guargummi mit Cᵢ_₆-Hydroxyalkylgruppen modifiziert ist.

5. Zusammensetzung nach Anspruch 4, dadurch gekennzeichnet, daß die Hydroxyalkylgruppen unter Hydroxymethylgruppen, Hydroxyethylgruppen, Hydroxypropylgruppen und Hydroxybutylgruppen ausgewählt sind.

6. Zusammensetzung nach einem der Ansprüche 3 bis 5, dadurch gekennzeichnet, daß das nichtionische Guargummi einen Oxyalkylierungsgrad aufweist, der im Bereich von 0,4 bis 1,2 liegt.

7. Zusammensetzung nach Anspruch 1 oder 2, dadurch gekennzeichnet, daß das kationische Guargummi Trialkylammoniumgruppen enthält.

8. Zusammensetzung nach Anspruch 7, dadurch gekennzeichnet, daß die Trialkylammoniumgruppen unter Trimethylammoniumgruppen und Triethylammoniumgruppen ausgewählt sind.

9. Zusammensetzung nach Anspruch 7 oder 8, dadurch gekennzeichnet, daß die Trialkylammoniumgruppen weniger als 30 Gew.-%, bezogen auf das Gesamtgewicht des modifizierten Guargummis, ausmachen.

10. Zusammensetzung nach Anspruch 9, dadurch gekennzeichnet, daß die Trialkylammoniumgruppen 5 bis 20 Gew.- %, bezogen auf das Gesamtgewicht des Guargummis, ausmachen.

11. Zusammensetzung nach einem der Ansprüche 7 bis 10, dadurch gekennzeichnet, daß das Guargummi mit 2,3-Epoxypropyltrimethylammoniumchlorid modifiziert ist.

12. Zusammensetzung nach einem der vorhergehenden Ansprüche, dadurch gekennzeichnet, daß die mit Oxyalkylengruppen modifizierten Silicone unter den Verbindungen der allgemeinen Formeln (I), (II), (III) und (IV) ausgewählt sind: 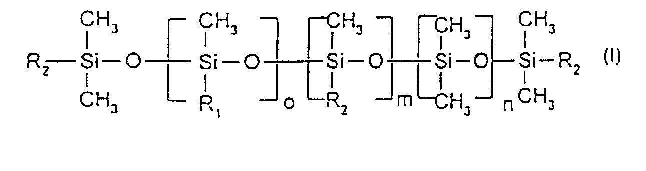 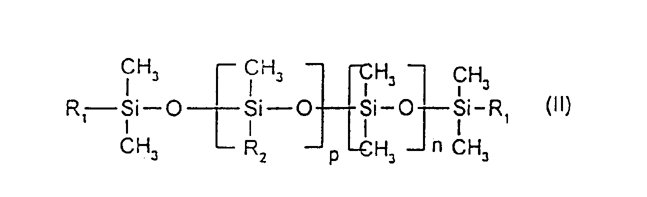 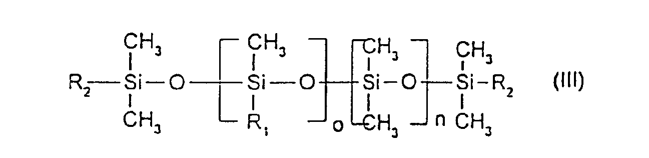 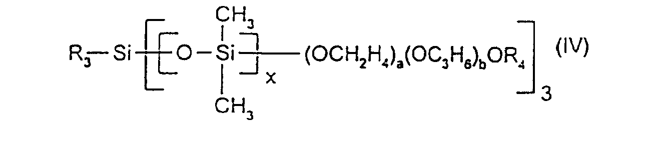 worin bedeuten:
- Ri C₁₋₃₀-Alkyl oder Phenyl,
- R₂ (CH₂)c(-C₂H₄O)ₐ(-C₃H₆O)_{b}-R₅,
- R₃ und R₄ C₁₋₁₂-Alkyl,
- R₅ Wasserstoff, C₁₋₁₂-Alkyl, C₁₋₆-Alkoxy, Hydroxy, -SOₛM, -OCOR₆, C₁₋₆-Aminoalkoxy, das gegebenenfalls an der Aminogruppe substituiert ist, C₁₋₆-Aminoacyl, das gegebenenfalls an der Aminogruppe substituiert ist, - NHCH₂CH₂COOM, N(CH₂CH₂COOM)₂, Aminoalkyl, das gegebenenfalls an der Aminogruppe und an der Alkylkette substituiert ist, C₁₋₃₀-Carboxyacyl, eine Phosphonogruppe, die gegebenenfalls mit einer oder zwei substituierten Aminoalkylgruppen substituiert ist, -CO(CH₂)_{d}COOM, -OCOCHR₇(CH₂)_{d}COOM,-NHCO(CH₂)_{d}OH oder NH₃Y,
wobei
M ein Wasserstoffatom, Na, K, Li, NH₄ oder ein organisches Amin,
R₆ eine G₁₋₃₀-Alkylgruppe,
R₇ Wasserstoff oder S0₃M und
d eine Zahl im Bereich von 1 bis 10 darstellen,
- m 0 bis 20,
- n 1 bis 100,
- o 0 bis 20,
- p 1 bis 20,
- a 0 bis 50,
b 0 bis 50,
wobei a + b größer oder gleich 1 ist,
- c 0 bis 4,
x 1 bis 100 und
- Y ein einwertiges anorganisches oder organisches Anion.

13. Zusammensetzung nach einem der Ansprüche 1 bis 12, dadurch gekennzeichnet, daß die Silicone der allgemeinen Formel (I) oder (11) entsprechen.

14. Zusammensetzung nach Anspruch 13, dadurch gekennzeichnet, daß R₅ ein Wasserstoffatom bedeutet.

15. Zusammensetzung nach einem der Ansprüche 1 bis 14, dadurch gekennzeichnet, daß die Guargummen in Prozentanteilen vorliegen, die im Bereich von etwa 0,01 bis 5 Gew.-%, bezogen auf das Gesamtgewicht der Zusammensetzung, und vorzugsweise im Bereich von 0,1 bis 2,5 % liegen.

16. Zusammensetzung nach einem der Ansprüche 1 bis 15, dadurch gekennzeichnet, daß die oxyalkylierten Silicone in Prozentanteilen vorliegen, die im Bereich von etwa 0,01 bis 5 Gew.-%, bezogen auf das Gesamtgewicht der Zusammensetzung, und vorzugsweise im Bereich von 0,1 bis 2,5 % liegen.

17. Zusammensetzung nach einem der Ansprüche 1 bis 16, dadurch gekennzeichnet, daß das kosmetisch akzeptable Medium aus Wasser Oder einem Gemisch von Wasser und kosmetisch akzeptablen Lösungsmitteln besteht, die ausgewählt sind unter einwertigen Alkoholen, mehrwertigen Alkoholen, Glykolethern und Fettsäureestern, die alleine oder im Gemisch verwendet werden können.

18. Zusammensetzung nach einem der Ansprüche 1 bis 17, dadurch gekennzeichnet, daß sie ferner grenzflächenaktive Mittel, Verdickungsmittel, Konservierungsmittel, Maskierungsmittel, reizlindernde Mittel, Parfüms, Färbemittel, Mittel zum Modifizieren der Viskosität, Schaummodifikatoren, Schaumbildner, Schaumstabilisatoren, Perlglanzmittel, Hydratisierungsmittel, Mittel gegen Schuppen, Mittel gegen Seborrhoe, Sonnenschutzfilter, Proteine, Vitamine, Weichmacher, Hydroxysäuren, Elektrolyte, Treibmittel, Parfüms oder Konditioniermittel enthält.

19. Zusammensetzung nach einem der Ansprüche 1 bis 18, dadurch gekennzeichnet, daß sie als Gel, Milch, Creme, Gelcreme, Spray, mehr oder weniger dickflüssige Lotion oder als Schaum vorliegt.

20. Verfahren zur nicht reinigenden kosmetischen Behandlung von Keratinsubstanzen, dadurch gekennzeichnet, daß eine in einem der Ansprüche 1 bis 19 definierte Zusammensetzung auf Keratinsubstanzen aufgetragen wird, und dann gegebenenfalls, nach einer eventuellen Einwirkungszeit, ausgespült wird.

21. Verfahren nach Anspruch 20, dadurch gekennzeichnet, daß die Keratinsubstanzen unter den Haaren und den Wimpern ausgewählt sind.

## Patentansprüche (Patentansprüche für folgende(n) Vertragsstaat(en) : FR)

1. Nicht reinigende kosmetische Zusammensetzung, dadurch gekennzeichnet, daß sie in einem kosmetisch akzeptablen Medium mindestens ein Guargummi und mindestens ein mit Oxyalkylengruppen modifiziertes Silicon in einem Gewichtsverhältnis Guargummi/Silicon kleiner oder gleich 5 enthält, mit Ausnahme der Kombination eines geradkettigen Polysiloxan-Polyoxyalkylen-Blockcopolymers mit einem Guargummi mit Hydroxypropylgruppen, der mit 2,3-Epoxypropyltrimethylammoniumchlorid quaternisiert ist.

2. Zusammensetzung nach Anspruch 1, dadurch gekennzeichnet, daß das Guargummi unter nichtionischen oder kationischen Guargummen ausgewählt ist.

3. Zusammensetzung nach Anspruch 2, dadurch gekennzeichnet, daß das Guargummi unter nichtionischen Guargummen ausgewählt ist.

4. Zusammensetzung nach Anspruch 3, dadurch gekennzeichnet, daß das nichtionische Guargummi mit Cₗ₋₆-Hydroxyalkylgruppen modifiziert ist.

5. Zusammensetzung nach Anspruch 4, dadurch gekennzeichnet, daß die Hydroxyalkylgruppen unter Hydroxymethylgruppen, Hydroxyethylgruppen, Hydroxypropylgruppen und Hydroxybutylgruppen ausgewählt sind.

6. Zusammensetzung nach einem der Ansprüche 3 bis 5, dadurch gekennzeichnet, daß das nichtionische Guargummi einen Oxyalkylierungsgrad aufweist, der im Bereich von 0,4 bis 1,2 liegt.

7. Zusammensetzung nach Anspruch 1 oder 2, dadurch gekennzeichnet, daß das kationische Guargummi Trialkylammoniumgruppen enthält.

8. Zusammensetzung nach Anspruch 7, dadurch gekennzeichnet, daß die Trialkylammoniumgruppen unter Trimethylammoniumgruppen und Triethylammoniumgruppen ausgewählt sind.

9. Zusammensetzung nach Anspruch 7 oder 8, dadurch gekennzeichnet, daß die Trialkylammoniumgruppen weniger als 30 Gew.-%, bezogen auf das Gesamtgewicht des modifizierten Guargummis, ausmachen.

10. Zusammensetzung nach Anspruch 9, dadurch gekennzeichnet, daß die Trialkylammoniumgruppen 5 bis 20 Gew.- %, bezogen auf das Gesamtgewicht des modifizierten Guargummis, ausmachen.

11. Zusammensetzung nach einem der Ansprüche 7 bis 10, dadurch gekennzeichnet, daß das Guargummi mit 2,3-Epoxypropyltrimethylammoniumchlorid modifiziert ist.

12. Zusammensetzung nach einem der vorhergehenden Ansprüche, dadurch gekennzeichnet, daß die mit Oxyalkylengruppen modifizierten Silicone ausgewählt sind unter den Verbindungen der allgemeinen Formeln (I), (11), (111) und (IV): 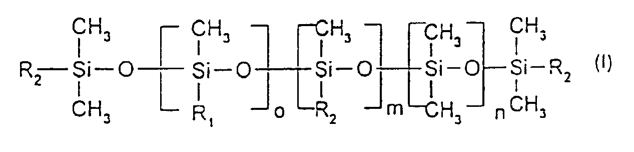 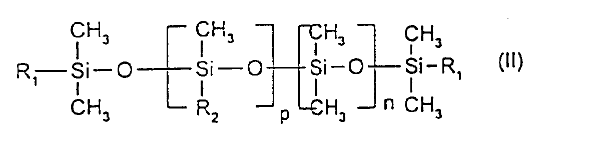 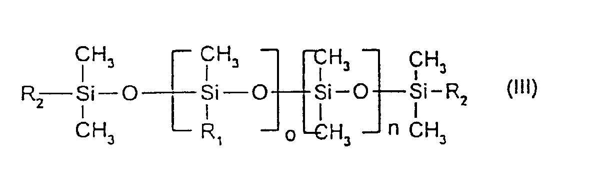 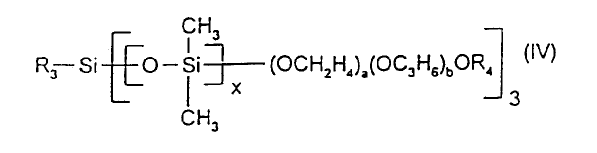 worin bedeuten:
- R₁ C₁₋₃₀-Alkyl oder Phenyl,
- R₂ (CH₂)_{c}(-C₂H₄O)ₐ(-C₃H₆O)_{b}-R₅,
- R₃ und R₄ C₁₋₁₂-Alkyl,
- R₅ Wasserstoff, C₁₋₁₂-Alkyl, C₁₋₆-Alkoxy, Hydroxy, -SO₃M, - OCOR₆, C₁₋₆-Aminoalkoxy, das gegebenenfalls an der Aminogruppe substituiert ist, C₁₋₆-Aminoacyl, das gegebenenfalls an der Aminogruppe substituiert ist, - NHCH₂CH₂COOM, N(CH₂CH₂COOM)₂, Aminoalkyl, das gegebenenfalls an der Aminogruppe und an der Alkylkette substituiert ist, C₁₋₃₀-Carboxyacyl, eine Phosphonogruppe, die gegebenenfalls mit einer oder zwei substituierten Aminoalkylgruppen substituiert ist, -CO(CH₂)_{d}COOM, -OCOCHR₇(CH₂)_{d}COOM,-NHCO(CH₂)_{d}OH oder NH₃Y,
wobei
M ein Wasserstoffatom, Na, K, Li, NH₄ oder ein organisches Amin,
R₆ eine C₁₋₃₀-Alkylgruppe,
R₇ Wasserstoff oder SO₃M und
d eine Zahl im Bereich von 1 bis 10 darstellen,
- m 0 bis 20,
n 1 bis 100,
- o 0 bis 20,
p 1 bis 20,
- a 0 bis 50,
- b 0 bis 50,
wobei a + b größer oder gleich 1 ist,
- c 0 bis 4,
- x 1 bis 100 und
Y ein einwertiges anorganisches oder organisches Anion.

13. Zusammensetzung nach einem der Ansprüche 1 bis 12, dadurch gekennzeichnet, daß die Silicone der allgemeinen Formel (I) oder (II) entsprechen.

14. Zusammensetzung nach Anspruch 13, dadurch gekennzeichnet, daß R₅ ein Wasserstoffatom bedeutet.

15. Zusammensetzung nach einem der Ansprüche 1 bis 14, dadurch gekennzeichnet, daß die Guargummen in Prozentanteilen vorliegen, die im Bereich von etwa 0,01 bis 5 Gew.-%, bezogen auf das Gesamtgewicht der Zusammensetzung, und vorzugsweise im Bereich von 0,1 bis 2,5 % liegen.

16. Zusammensetzung nach einem der Ansprüche 1 bis 15, dadurch gekennzeichnet, daß die oxyalkylierten Silicone in Prozentanteilen vorliegen, die im Bereich von etwa 0,01 bis 5 Gew.-%, bezogen auf das Gesamtgewicht der Zusammensetzung, und vorzugsweise im Bereich von 0,1 bis 2,5 % liegen.

17. Zusammensetzung nach einem der Ansprüche 1 bis 16, dadurch gekennzeichnet, daß das kosmetisch akzeptable Medium aus Wasser oder einem Gemisch von Wasser und kosmetisch akzeptablen Lösungsmitteln besteht, die ausgewählt sind unter einwertigen Alkoholen, mehrwertigen Alkoholen, Glykolethern und Fettsäureestern, die alleine oder im Gemisch verwendet werden können.

18. Zusammensetzung nach einem der Ansprüche 1 bis 17, dadurch gekennzeichnet, daß sie auch grenzflächenaktive Mittel, Verdickungsmittel, Konservierungsmittel, Maskierungsmittel, reizlindernde Mittel, Parfüms, Färbemittel, Mittel zum Modifizieren der Viskosität, Schaummodifikatoren, Schaumbildner, Schaumstabilisatoren, Perlglanzmittel, Hydratisierungsmittel, Mittel gegen Schuppen, Mittel gegen Seborrhoe, Sonnenschutzfilter, Proteine, Vitamine, Weichmacher, Hydroxysäuren, Elektrolyte, Treibmittel, Parfüms oder Konditioniermittel enthält.

19. Zusammensetzung nach einem der Ansprüche 1 bis 18, dadurch gekennzeichnet, daß sie als Gel, Milch, Creme, Gelcreme, Spray, mehr oder weniger dickflüssige Lotion oder als Schaum vorliegt.

20. Verfahren zur nicht reinigenden kosmetischen Behandlung von Keratinsubstanzen, dadurch gekennzeichnet, daß eine in einem der Ansprüche 1 bis 19 definierte Zusammensetzung auf Keratinsubstanzen aufgetragen wird, und dann gegebenenfalls, nach einer eventuellen Einwirkungszeit, ausgespült wird.

21. Verfahren nach Anspruch 20, dadurch gekennzeichnet, daß die Keratinsubstanzen unter den Haaren und den Wimpern ausgewählt sind.
